(19) Europäisches Patentamt
European Patent Office
Office européen des brevets

(11) Veröffentlichungsnummer: **0 074 005**
**B1**

(12) # EUROPÄISCHE PATENTSCHRIFT

(45) Veröffentlichungstag der Patentschrift: **02.05.85**

(21) Anmeldenummer: **82107700.5**

(22) Anmeldetag: **23.08.82**

(51) Int. Cl.⁴: **A 01 N 43/36,** A 01 N 43/40, A 01 N 43/84, C 07 D 295/02, C 07 D 211/14, C 07 D 265/30, C 07 D 295/06 // C07C47/21, C07C25/24, C07C33/48, C07C33/46, C07C21/24

(54) **Phenylpropylammoniumsalz enthaltende Fungizide und Verfahren zur Bekämpfung von Pilzen.**

(30) Priorität: **29.08.81 DE 3134220**
**09.09.81 DE 3135592**

(43) Veröffentlichungstag der Anmeldung: **16.03.83 Patentblatt 83/11**

(45) Bekanntmachung des Hinweises auf die Patenterteilung: **02.05.85 Patentblatt 85/18**

(84) Benannte Vertragsstaaten: **AT BE CH DE FR GB IT LI NL SE**

(56) Entgegenhaltungen:
**EP-A-0 002 604**
**EP-A-0 007 479**
**EP-A-0 031 114**
**LU-A-79 385**

(73) Patentinhaber: **BASF Aktiengesellschaft, Carl-Bosch-Strasse 38, D-6700 Ludwigshafen (DE)**

(72) Erfinder: **Buschmann, Ernst, Dr., Georg-Ludwig-Krebs-Strasse 10, D-6700 Ludwigshafen (DE)**
Erfinder: **Zeeh, Bernd, Dr., Thorwaldsenstrasse 5, D-6700 Ludwigshafen (DE)**
Erfinder: **Pommer, Ernst-Heinrich, Dr., Berliner Platz 7, D-6703 Limburgerhof (DE)**
Erfinder: **Ammermann, Eberhard, Dr., Sachsenstrasse 3, D-6700 Ludwigshafen (DE)**

**EP 0 074 005 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft quartäre Phenylpropylammoniumsalze, diese enthaltende Fungizide und Verfahren zur Bekämpfung von Pilzen.

Es ist bekannt, N-Trichlormethylthiotetrahydrophthalimid als Fungizid zu verwenden („Chemical Week", June 21, 1972, S. 46). Es ist ferner bekannt, N-arylpropylsubstituierte cyclische Amine (EP-A1 Nr. 7479) oder N-aryl-2-methyl-1-propylsubstituierte Morpholine (LU-A Nr. 79385) als Fungizide zu verwenden.

Es wurde nun gefunden, dass Phenylpropylammoniumsalze der Formel I'

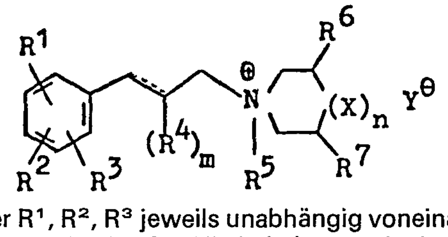 (I')

in der $R^1$, $R^2$, $R^3$ jeweils unabhängig voneinander Wasserstoff, $C_1$-$C_8$-Alkyl, halogensubstituiertes $C_1$-$C_4$-Alkyl, ggf. durch Halogen oder $C_1$-$C_4$-Alkyl substituiertes Phenyl, ggf. durch Halogen substituiertes Benzyl, 2,4,6-Trimethylbenzyl, $C_3$-$C_7$-Cycloalkyl, $C_1$-$C_4$-Alkoxy, $C_2$-$C_4$-Acyl oder Halogen, $R^4$ Wasserstoff, $C_1$-$C_6$-Alkyl, $C_3$-$C_4$-Alkenyl, $C_1$-$C_3$-Alkoxy, $R^5$ $C_1$-$C_4$-Alkyl, $C_2$-$C_4$-Alkenyl, Propargyl oder ggf. durch Halogen oder $C_1$-$C_4$-Alkyl substituiertes Benzyl, $R^6$ und $R^7$ unabhängig voneinander Wasserstoff, Methyl, Ethyl, Propyl, $CH_2OH$, OH und X $CH_2$, O, S, C=O, $(CH_2)_2$ oder $CH_2CH-R^8$ bedeutet,
wobei $R^8$ Alkyl bedeutet und m 1, 2, n 0 und 1 und $Y^\ominus$ das Anion einer nichtphytotoxischen Säure bedeutet,
eine gute fungizide Wirksamkeit zeigen.

Es wurden ferner neue Phenylpropylammoniumsalze der Formel I

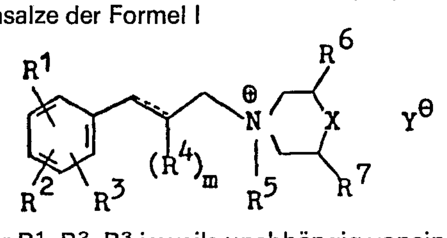 (I)

in der $R^1$, $R^2$, $R^3$ jeweils unabhängig voneinander Wasserstoff, $C_1$-$C_8$-Alkyl, halogensubstituiertes $C_1$-$C_4$-Alkyl, ggf. durch Halogen oder $C_1$-$C_4$-Alkyl substituiertes Phenyl, ggf. durch Halogen substituiertes Benzyl, 2,4,6-Trimethylbenzyl, $C_3$-$C_7$-Cycloalkyl, $C_1$-$C_4$-Alkoxy, $C_2$-$C_4$-Acyl oder Halogen $R^4$ Wasserstoff, $C_1$-$C_6$-Alkyl, $C_3$-$C_4$-Alkenyl, $C_1$-$C_3$-Alkoxy, $R^5$ $C_1$-$C_4$-Alkyl, $C_4$-Alkenyl, Propargyl oder ggf. durch Halogen oder $C_1$-$C_4$-Alkyl substituiertes Benzyl, $R^6$ und $R^7$ unabhängig voneinander Wasserstoff, Methyl, Ethyl, Propyl, $CH_2OH$, OH und X $CH_2$, O, S, C=O oder
$\overset{|}{C}H_2\overset{|}{C}H-R^8$ bedeutet,
wobei $R^8$ Alkyl bedeutet und m 1 oder 2, und $Y^\ominus$ das Anion einer nichtphytotoxischen Säure bedeutet,
ausser N-n-Butyl-3-(3-ethoxyphenyl)propylpiperidiniumoxalat gefunden.

$R^1$, $R^2$, $R^3$ bedeuten beispielsweise $C_1$-$C_8$-Alkyl (Methyl, Ethyl, n-Propyl, Isopropyl, n-Butyl, Isobutyl, tert.-Butyl, n-Pentyl, tert.-Amyl, 1,1-Dimethylbutyl, 1,1-Dimethylpentyl, 1,1-Dimethylhexyl, 1,1-Diethylethyl, 1,1,2-Trimethylpropyl), $C_1$-$C_4$-Haloalkyl (2-Chlor-1,1-dimethylethyl, 2-Fluor-1,1-dimethylethyl, 2-Brom-1,1-dimethylethyl, Trichlormethyl, Trifluormethyl), $C_3$-$C_7$-Cycloalkyl (Cyclopropyl, Cyclopentyl, Cyclohexyl, Cycloheptyl), Phenyl, Halophenyl, $C_1$-$C_4$-Alkylphenyl (4-tert.-Butylphenyl, 4-Chlorphenyl, Benzyl), Halobenzyl (4-Chlorbenzyl), 2,4,6-Trimethylbenzyl, $C_1$-$C_4$-Alkoxy, Methoxy, Ethoxy, tert.-Butoxy, $C_2$-$C_4$-Acyl (Acetyl, Propionyl, Butyryl), Fluor, Chlor, Brom, Jod, Wasserstoff;

$R^4$ bedeutet beispielsweise $C_1$-$C_6$-Alkyl (Methyl, Ethyl, n-Propyl, Isopropyl, n-Butyl, Isobutyl, tert.-Butyl, n-Pentyl, n-Hexyl), $C_3$-$C_4$-Alkenyl (Propen-1-yl, Buten-1-yl), $C_1$-$C_3$-Alkoxy (Methoxy, Ethoxy, Propoxy);

$R^5$ bedeutet beispielsweise $C_1$-$C_4$-Alkyl (Methyl, Ethyl, n-Propyl, n-Butyl, Isobutyl), $C_4$-Alkenyl (2-Butenyl, 2-Methylallyl), Propargyl, Benzyl, Halobenzyl (4-Chlorbenzyl, 4-Fluorbenzyl, 4-Brombenzyl, 4-Jodbenzyl), $C_1$-$C_4$-Alkylbenzyl (4-Methylbenzyl, 4-tert.-Butylbenzyl, 2,4-Dichlorbenzyl, 2,6-Dichlorbenzyl, 2,3,6-Trichlorbenzyl, 2,3,4-Trichlorbenzyl, 3,4-Dichlorbenzyl);

$R^6$ und $R^7$ bedeuten beispielsweise Wasserstoff, Methyl, Ethyl, Propyl, $CH_2OH$, OH;

$Y^\ominus$ bedeutet beispielsweise $Cl^\ominus$, $Br^\ominus$, $J^\ominus$, $NO_3^\ominus$, ½ $(SO_4^{2-})$, $CH_3C_6H_5SO_3^\ominus$.

Die dem aromatischen Ring ggf. benachbarte Doppelbindung kann Z- oder E-konfiguriert sein. Die Substituenten $R^6$ und $R^7$ können zueinander cis- oder trans-konfiguriert sein. Auch diese Isomere können in den fungiziden Mitteln enthalten sein.

Die obengenannten Phenylpropylammoniumsalze sind Wirkstoffe in Fungiziden. Sie zeigen eine gute fungizide Wirkung.

Die Phenylpropylammoniumsalze sind z. T. bekannt aus DE-OS Nr. 2952382.

Verbindungen, die noch nicht beschrieben sind, sind leicht zugänglich aus Phenylpropylaminen der Formel II

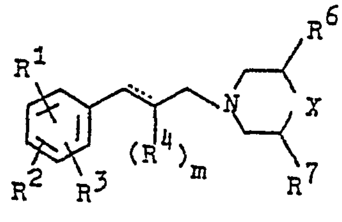 (II)

in der $R^1$, $R^2$, $R^3$, $R^4$, $R^6$, $R^7$, m und X die obengenannten Bedeutungen haben (teilweise bekannt aus DE-OS Nr. 2752096). Diese tertiären Amine lassen sich mit Quaternierungsmitteln $R^5Y$ zu den Endverbindungen umsetzten. Als Quaternierungsmittel kommen ausser den Alkyl-, Alkenyl-, Alkinyl-($R^5$)-halogeniden z. B. in Betracht: Dimethylsulfat, Diethylsulfat, Sulfonsäureester der Formel $RSO_3R^5$, wobei R $C_1$- bis $C_7$-Alkyl oder durch

Halogen oder Alkyl substituiertes Phenyl oder Aralkyl bedeuten kann.

Alternativ können die erfindungsgemässen quartären Ammoniumsalze hergestellt werden durch Umsetzung von Aminen der Formel

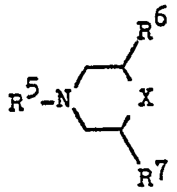

mit Phenylpropylhalogeniden der Formel III

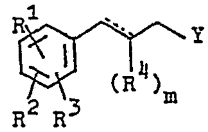

(III)

in der $R^1$, $R^2$, $R^3$, $R^4$, $R^5$, $R^6$, $R^7$, X und m die obengenannten Bedeutungen haben und Y, Cl, Br oder J bedeutet, die teilweise aus der DE-OS Nr. 2752096 bekannt sind.

Das folgende Schema beschreibt, wie die Verbindungen der Formeln II und III mit allgemein bekannten Reaktionen hergestellt werden können.

Die Herstellung der Aldehyde IV ist beschrieben von B. Zeeh und E. Buschmann in „Liebigs Ann. Chem.“, 1979, S. 1585.

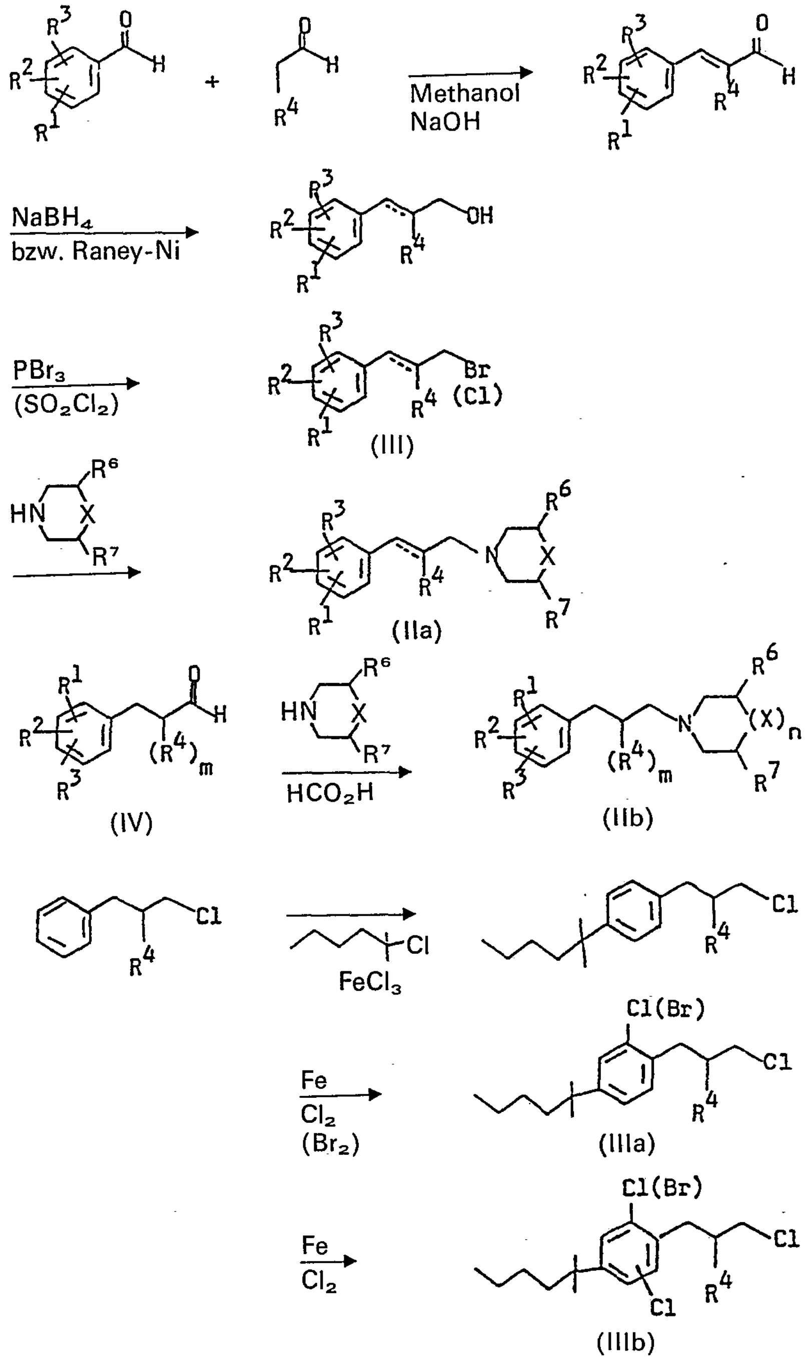

Die Phenylpropylammoniumsalze erhält man, indem man ein Phenylpropylamin der Formel II mit einem Alkylierungsmittel der Formel $R^5Y$, wobei $R^5$ und Y die obengenannten Bedeutungen haben, umsetzt.

Alternativ erhält man die Wirkstoffe, indem man ein Phenylpropylhalogenid der Formel III mit tertiären Aminen der Formel V

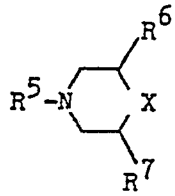

(V)

in der $R^5$, $R^6$, $R^7$ und X die obengenannten Bedeutung haben, umsetzt.

Die Umsetzung der Phenylpropylamine II mit $R^5Y$ und die Umsetzung der Phenylpropylhalogenide III mit den tertiären Aminen V erfolgt beispielsweise bei einer Temperatur von 10 bis 150° C in Anwesenheit oder Abwesenheit eines Lösungsmittels, z. B. Ethanol, Methanol, $CHCl_3$, $CH_2Cl_2$, Aceton, Cyclohexanon, THF, Acetonitril, Dimethylformamid oder Essigester bei Normaldruck oder erhöhtem Druck.

Die folgenden Beispiele erläutern die Herstellung der Verbindungen.

*Beispiel 1:*

a) *2-n-Pentyl-3-(2,4-dichlorphenyl)acrolein I*

Zu einer Lösung von 525 g 2,4-Dichlorbenzaldehyd und 12 g NaOH in 1,5 l MeOH werden innerhalb von 6 h 342 g Heptanal zugetropft. Man lässt 1 h nachreagieren, säuert an mit Eisessig, engt nach weiteren 15 h ein und nimmt den Rückstand mit $CH_2Cl_2/H_2O$ auf. Die organische Phase wird mit Wasser gewaschen, über $Na^2SO_4$ getrocknet und eingeengt. Destillation des Rückstandes ergeben 498 g I; Sdp. 152-160° C/0,2 mbar.

b) *2-n-Pentyl-3-(2,4-dichlorphenyl)allylalkohol II*

Zu einer Lösung von 150 g I in 1,5 MeOH werden portionsweise 54,5 g $NaBH_4$ zugegeben. Man erwärmt 1 h zum Rückfluss, engt ein, gibt 1 h 2N-HCl zu, erwärmt 1 h zum Rückfluss und extrahiert mit $CH_2Cl_2$. Die organische Phase wird mit Wasser gewaschen, über $Na_2SO_4$ getrocknet und eingeengt. Destillation des Rückstandes ergibt 93 g II; Sdp. 156-160° C/0,2 mbar.

c) *2-n-Pentyl-3-(2,4-dichlorphenyl)allylbromid III*

Zur Lösung von 93 g II in 300 ml $CHCl_3$ werden bei 10° C 32,5 g $PBr_3$ zugetropft. Man rührt 15 h bei Raumtemperatur und giesst die $CHCl_3$-Lösung in Eiswasser. Die organische Phase wird abgetrennt, die wässerige Phase mit $CHCl_3$ extrahiert. Die vereinigten organischen Phasen werden mehrfach mit wässeriger $Na_2CO_3$-Lösung und mit Wasser gewaschen, über $Na_2SO_4$ getrocknet, eingeengt und destilliert. Man erhält 110 g III; Sdp. 140 bis 148° C/0,1 mbar.

d) *N-[2-n-Pentyl-3-(2,4-dichlorphenyl)prop-2-en-1-yl]pyrrolidin IV*

Eine Mischung von 50 g III und 31,7 g Pyrrolidin wird 5 h auf einem 150° C warmen Ölbad erwärmt. Das abgekühlte Rohprodukt wird in $CHCl_3$ gelöst, mit verd. NaOH and anschliessend mehrfach mit Wasser gewaschen. Die organische Phase wird über $Na_2SO_4$ getrocknet, eingeengt und destilliert. Man erhält 19 g IV; Sdp. 148-150° C/0,1 mbar.

e) *N-[2-n-Pentyl-3-(2,4-dichlorphenyl)prop-2-en-1-yl]-N-allylpyrrolidiniumbromid V*

19 g IV und 14,5 g Allylbromid in 200 ml Eisessig werden 5 h zum Rückfluss erwärmt. Man kühlt ab und verreibt das flüssig ausgefallene Produkt mit Essigester. Das dabei kristallisierte Produkt wird abgesaugt, mit Essigester gewaschen und im Vakuum getrocknet. Man erhält 14 g V; Fp. 109° C (Verbindung Nr. 3a).

*Beispiel 2:*

a) *1-[4-tert-Butylphenyl]-2-[4-(3-chlor-2-methylpropyl)phenyl]propan-2-ol VI*

Zu einer Grignard-Suspension, hergestellt aus 182,5 g 4-tert.-Butylbenzylchlorid und 26 g Mg in 400 ml $Et_2O$, wird zugetropft 168,4 g 3-(4-Acetylphenyl)-2-methylpropylchlorid in 100 ml $Et_2O$. Nach Ende des Zutropfens wird noch 2 h zum Rückfluss erwärmt. Man hydrolysiert mit eisgekühlter verdünnter wässeriger HCl bis pH = 2. Die organischen Produkte werden mit Ether extrahiert. Die organische Phase wird mit Wasser gewaschen, über $Na_2SO_4$ getrocknet und eingeengt. Destillation des Rückstandes ergibt 163,4 g IV; Sdp. 190° C/0,1 mbar.

b) *N-{3-[-(1-4-t-Butylphenyl)propan-2-ol-2-yl]phenyl-2-methylpropyl}-2,6-cis-dimethylmorpholin VIII*

Eine Mischung von 164 g VI und 159 g 2,6-cis-Dimethylmorpholin wird 6 h bei 150° C gerührt. Das Rohprodukt wird in $CHCl_3$ gelöst, mit verdünnter wässeriger NaOH und anschliessend mit Wasser gewaschen. Man trocknet über $Na_2SO_4$, engt ein und destilliert. Ausbeute 147 g VII; Sdp. 214-218° C/0,3 mbar.

c) *N-{3-[4-(1-4-t-Butylphenyl)propan-2-yl]-phenyl-2-methylpropyl}-2,6-cis-dimethylmorpholin VIII*

Eine Lösung von 50 g VII in 1 l Eisessig und 30 ml $H_2SO_4$ konz. werden in Gegenwart von 5 g 5%iger Pd/C bei 5 bar und Raumtemperatur bis zur Druckkonstanz hydriert. Man filtriert den Katalysator ab und stellt mit verdünnter wässeriger NaOH alkalisch. Das Rohprodukt wird mit $CH_2Cl$ extrahiert und mit $H_2O$ gewaschen. Man trocknet über $Na_2SO_4$, engt ein und destilliert; Sdp. 200-202° C/0,3 mbar, 40 g Ausbeute.

d) *N-{3-[4-(1-4-t-Butylphenyl)propan-2-yl]-phenyl-2-methylpropyl}-N-methyl-2,6-cis-dimethylmorpholiniumbromid IX*

Zu einer Lösung von 38 g $CH_3Br$ in 200 ml

$CH_3CN$ werden 42 g VIII in 200 ml $CH_3CN$ zugegeben. Nach 15 h bei Raumtemperatur wird eingeengt. Der Rückstand kristallisiert beim Verreiben mit Ether. Das Produkt wird abgesaugt, mit Ether gewaschen und im Vakuum getrocknet. 36 g Ausbeute; Fp. 210° C (Zersetzung) (Verbindung Nr. 65).

*Beispiel 3:*

a) *N-[3-(2,4-Dichlorphenyl)-2-methyl-2-prop-2-en-1-ylpropyl]pyrrolidin X*

Zu 45,8 g Pyrrolidin werden unter Eiskühlung 149 g Ameisensäure zugetropft. Nach Zugabe von 181 g 3-(2,4-Dichlorphenyl)-2-methyl-2-propen-1-yl-propionaldehyd wird 12 h auf 100° C erwärmt. Man dampft im Vakuum ein, stellt mit 25%iger NaOH alkalisch, extrahiert mit Ether, trocknet über KOH, engt ein und destilliert. Ausbeute: 95 g X; Sdp. 130-138° C/0,1 mbar.

b) *N-Allyl-N-[3-(2,4-Dichlorphenyl)-2-methyl-2-prop-2-en-1-ylpropyl]pyrrolidiniumbromid XI*

Eine Lösung von 40 g X und 31,5 g Allylbromid in 300 ml Essigester wird 5 h zum Rückfluss erwärmt. Das ausgefallene Produkt wird abgesaugt, mit Essigester gewaschen und im Vakuum getrocknet. 15 g Ausbeute; Fp. 174° C (Verbindung Nr. 56).

*Beispiel 4:*

a) *3-[4-(1,1-Dimethylpentyl)phenyl]-2-methylpropylchlorid XII*

Zu einem Gemisch aus 428 g 3-Phenyl-2-methylpropylchlorid und 40,6 g $FeCl_3$ werden bei 35° C 342 g 1,1-Dimethylpentylchlorid zugetropft. Man rührt 7 h bei 50° C und 14 h bei Raumtemperatur. Das Rohprodukt wird in 2 l $CHCl_3$ gelöst, mit verdünnter HCl und Wasser gewaschen, über $Na_2CO_3$ getrocknet, eingeengt und destilliert. Man erhält 500 g XII; Sdp. 126-132° C/0,2 mbar.

b) *3-[2-Brom-4-(1,1-dimethylphenyl)phenyl]-2-methylpropylchlorid XIII*

Zu einer Mischung aus 300 g XII und 3 g Fe-Pulver werden 180 g $Br_2$ bei Raumtemperatur zugetropft. Man rührt 14 h bei Raumtemperatur, löst das Rohprodukt in $CH_2Cl_2$, wäscht mit Wasser, trocknet über $Na_2SO_4$ und destilliert. Man erhält 220 g XIII; Sdp. 160° C/0,1 mbar.

c) *N-{3-[2-Brom-4-(1,1-dimethylpentyl)phenyl]-2-methylpropyl}piperidin XIV*

Eine Mischung von 50 g XIII und 38,3 g Piperidin wird 7 h auf 150° C erwärmt. Nach Abkühlen wird mit $CHCl_3$ aufgenommen, mit verd. NaOH und anschliessend mit Wasser gewaschen, über $Na_2SO_4$ getrocknet und eingeengt. Destillation des Rückstandes ergibt 27 g XIV; Sdp. 170-171° C/0,1 mbar.

d) *N-{3-[2-Brom-4-(1,1-dimethylpentyl)phenyl]-2-methylpropyl}-N-methylpiperidiniumbromid XV*

Zu einer Lösung von 16,4 g $CH_3Br$ in 200 ml $CH_3CN$ gibt man 17 g XIV in 100 ml $CH_3CN$. Nach 14 h Raumtemperatur wird eingeengt. Der kristalline Rückstand wird im Ether verrieben, abgesaugt und im Vakuum getrocknet. Ausbeute: 15 g; Fp. 178° C (Verbindung Nr. 78).

*Beispiel 5:*

a) *3-[2,6-Dichlor-4-(1,1-dimethylpentyl)phenyl]-2-methylpropylchlorid XVI*

Zu einer Suspension von 4 g Fe-Pulver in 500 g XII werden bei 10 bis 30° C 178 g $Cl_2$ eingegast. Die Bildung des zunächst mono-, später dichlorierten Produktes wird gaschromatographisch verfolgt. Eventuell wird noch zusätzlich $Cl_2$ eingegast. Man nimmt das Rohprodukt in $CH_2Cl_2$ auf, wäscht mit Wasser, trocknet über $Na_2SO_4$ und destilliert. Man erhält 390 g XVI; Sdp. 152° C/0,1 mbar.

Bei der oben beschriebenen Chlorierung entstehen als Nebenprodukte die isomeren 2,5- und 2,3-Dichlorphenylverbindungen.

b) *N-{3-[2,6-Dichlor-4-(1,1-dimethylpentyl)-phenyl]-2-methylpropyl}piperidin XVII*

Darstellung aus 150 g XVI und 115 g Piperidin. Herstellmethode wie Beispiel 4c. 125 g Ausbeute; Sdp. 177-185° C/0,3 mbar.

Im oben beschriebenen Piperidinderivat sind die entsprechenden 2,3- und 2,5-Dichlorphenylverbindungen als Nebenprodukte enthalten (s. Beispiel 5a).

c) *N-{3-[2,6-Dichlor-4-(1,1-dimethylpentyl)-phenyl]-2-methylpropyl}-N-(4-$CF_3$-benzyl)piperidiniumbromid XVIII*

Eine Lösung von 17 g XVII und 17,9 g p-$CF_3$-Benzylbromid in 300 ml Essigester wird 6 h zum Rückfluss erwärmt. Man kühlt ab, saugt das ausgefallene Produkt ab, wäscht mit Essigester und trocknet im Vakuum. Ausbeute 2 g; Fp. 206° C. Einengen der Mutterlauge, Verreiben des Rückstandes mit Essigester, absaugen, waschen und trocknen ergibt weitere 16 g XVIII; Fp. 204° C (Verbindung Nr. 86).

Im oben beschriebenen Salz sind die entsprechenden 2,3- und 2,5-Dichlorverbindungen als Nebenprodukte enthalten (s. Beispiel 5b).

*Beispiel 6:*

a) *2-n-Butyl-3-(2,3,4-trichlorphenyl)acrolein XIX*

Herstellung nach Vorschrift von Beispiel 1a aus 266 g 2,3,4-Trichlorbenzaldehyd und 127 g Hexanal. Ausbeute 210 g; Sdp. 181-182° C/0,2 mbar.

b) *2-n-Butyl-3-(2,3,4-trichlorphenyl)propan-1-ol XX*

Eine Suspension von 60 g Raney-Nickel in 435 g XIX und 1 l Methanol wird mit Stickstoff gespült. Anschliessend wird im Autoklaven bei 60 bis 70° C und 100 bar bis zur Druckkonstanz hydriert. Man saugt den Katalysator ab, engt ein und destilliert. Ausbeute 278 g XX; Sdp. 167° C/0,1 mbar.

c) *2-n-Butyl-3-(2,3,4-trichlorphenyl)propylchlorid XXI*

Zu 148 g Thionylchlorid werden 332 g XX zugetropft. Man rührt 14 h bei Raumtemperatur und 2 h bei 140° C. Destillation ergibt 278 g XXI; Sdp. 145-152° C/0,1 mbar.

d) *N-[2-n-Butyl-3-(2,3,4-trichlorphenyl)propyl]-pyrrolidin XXII*

Darstellung aus 140 g XXI und 95 g Pyrrolidin. Herstellungsmethode wie Beispiel 4c. 135 g Ausbeute; Sdp. 166-167° C/0,1 mbar.

e) *N-Allyl-N-[2-n-Butyl-3-(2,3,4-trichlorphenyl)propyl]pyrrolidiniumbromid XXIII*

Eine Lösung von 34,8 g XXII und 36,3 g Allylbromid in 250 ml Essigester wird 5 h zum Rückfluss erwärmt. Das Produkt fällt als Öl aus und wird mehrfach mit Essigester gewaschen und anschliessend im Vakuum von Lösungsmittelresten befreit. Ausbeute 32 g, gelb-braunes Harz (Verbindung Nr. 60).

In entsprechender Weise werden die folgenden Verbindungen hergestellt.

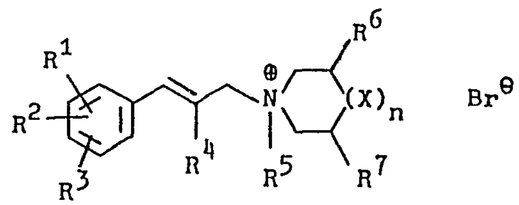

| Nr. | $R^1$ | $R^2$ | $R^3$ | $R^4$ | $R^5$ | $R^6$ | $R^7$ | $(X)_n$ | Y | Fp. (° C) |
|---|---|---|---|---|---|---|---|---|---|---|
| 1a | 2-Cl | 4-Cl | H | $CH_3$ | Allyl | H | H | — | Br | 110 |
| 2a | 2-Cl | 4-Cl | H | n-Pentyl | Allyl | H | H | $CH_2$ | Br | 148 |
| 3a | 2-Cl | 4-Cl | H | n-Pentyl | Allyl | H | H | — | Br | 109 |
| 4a | 2-Cl | 4-Cl | H | iso-Propyl | Allyl | H | H | — | Br | 75 |
| 5a | 2-Cl | 3-Cl | 4-Cl | n-Butyl | Allyl | H | H | — | Br | |
| 6a | 2-Cl | 3-Cl | 4-Cl | n-Butyl | $CH_3$ | H | H | — | | |
| 7a | 2-Cl | 3-Cl | 4-Cl | n-Butyl | 4-Cl-Benzyl | H | H | — | Br | |
| 8a | 2-Cl | 3-Cl | 4-Cl | n-Propyl | Allyl | H | H | — | Br | |
| 9a | 2-Cl | 3-Cl | 4-Cl | n-Propyl | $CH_3$ | H | H | — | Br | |
| 10a | 2-Cl | 3-Cl | 4-Cl | n-Propyl | 4-Cl-Benzyl | H | H | — | Br | |
| 11a | 4-iso-Propyl | H | H | $CH_3$ | $CH_3$ | H | H | — | Br | |

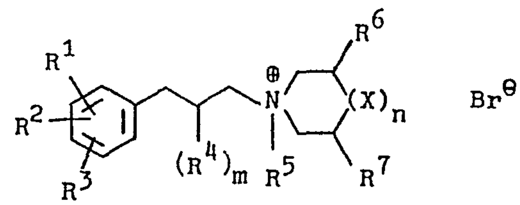

| Nr. | $R^1$ | $R^2$ | $R^3$ | $(R^4)_m$ | $R^5$ | $R^6$ | $R^7$ | $(X)_n$ | Y | Fp. (° C) |
|---|---|---|---|---|---|---|---|---|---|---|
| 1 | 4-t-Bu (tertiär Butyl) | H | H | $CH_3$ | Allyl | H | H | CH-t-Bu$CH_2$ | Br | 153 |
| 2 | 4-$CH(CH_3)_2$ | H | H | $CH_3$ | Allyl | H | H | CH-t-Bu$CH_2$ | Br | 152 |
| 3 | 4-t-Bu | H | H | $CH_3$ | $CH_3$ | H | H | CH-t-Bu$CH_2$ | Br | 216 |
| 4 | 4-$CH(CH_3)_2$ | H | H | $CH_3$ | $CH_3$ | H | H | CH-t-Bu$CH_2$ | Br | 190 |
| 5 | 4-t-Bu | H | H | $CH_3$ | $CH_3$ | $CH_3$ | $CH_3$ | O | Br | 184 |
| 6 | 4-t-Bu | H | H | $CH_3$ | Propargyl | $CH_3$ | $CH_3$ | O | Br | 82 |
| 7 | 4-t-Bu | H | H | $CH_3$ | Allyl | $CH_3$ | $CH_3$ | O | Br | 61 |
| 8 | 4-t-Bu | H | H | $CH_3$ | Ethyl | $CH_3$ | $CH_3$ | O | $C_2H_5SO_4^{\ominus}$ | 85 |
| 9 | 4-t-Bu | H | H | $CH_3$ | $CH_3$ | $CH_3$ | $CH_3$ | $CH_2$ | Br | 220 |
| 10 | 4-t-Bu | H | H | $CH_3$ | $C_2H_5$ | $CH_3$ | $CH_3$ | $CH_2$ | Br | 197 |
| 11 | 4-t-Bu | H | H | $CH_3$ | Allyl | $CH_3$ | $CH_3$ | $CH_2$ | Br | 80 |
| 12 | 4-t-Bu | H | H | $CH_3$ | Benzyl | $CH_3$ | $CH_3$ | $CH_2$ | Br | 224 |
| 13 | 4-t-Bu | H | H | $CH_3$ | 4-Cl-Benzyl | $CH_3$ | $CH_3$ | $CH_2$ | Br | 187 |
| 14 | 4-t-Bu | H | H | $CH_3$ | 4-F-Benzyl | $CH_3$ | $CH_3$ | $CH_2$ | Br | 209 |
| 15 | 4-t-Bu | H | H | $CH_3$ | $CH_3$ | $CH_3$ | $CH_3$ | O | $CH_3SO_4^{\ominus}$ | 130 |
| 16 | 4-t-Bu | H | H | $CH_3$ | $C_2H_5$ | $CH_3$ | $CH_3$ | O | $C_2H_5SO_4^{\ominus}$ | 72 |
| 17 | 4-t-Bu | H | H | $CH_3$ | Benzyl | $CH_3$ | $CH_3$ | O | Br | 196 |
| 18 | 4-t-Bu | H | H | $CH_3$ | 4-Br-Benzyl | $CH_3$ | $CH_3$ | O | Br | 198 |

| Nr. | $R^1$ | $R^2$ | $R^3$ | $(R^4)_m$ | $R^5$ | $R^6$ | $R^7$ | $(X)_n$ | Y | Fp. (°C) |
|---|---|---|---|---|---|---|---|---|---|---|
| 19 | 4-t-Bu | H | H | $CH_3$ | 4-F-Benzyl | $CH_3$ | $CH_3$ | O | Br | 194 |
| 20 | 4-t-Bu | H | H | $CH_3$ | 4-Cl-Benzyl | $CH_3$ | $CH_3$ | O | Br | 187 |
| 21 | 4-t-Bu | H | H | $CH_3$ | Allyl | $CH_3$ | $CH_3$ | O | Br | 94 |
| 22 | 2-Cl | 4-Cl | H | $CH(CH_3)_2$ | Allyl | H | H | — | Br | 123 |
| 23 | 2-Cl | 4-Cl | H | n-Propyl | Allyl | H | H | — | Br | Öl |
| 24 | 2-Cl | 4-Cl | 6-Cl | H | Allyl | H | H | — | Br | 115 |
| 25 | 4-Br | H | H | H | Allyl | H | H | — | Br | 126 |
| 26 | 4-Cl | H | H | $CH_3$ | Allyl | H | H | — | Br | Harz |
| 27 | 2-F | H | H | $CH_3$ | Allyl | H | H | — | Br | Harz |
| 28 | 2-Cl | 4-Cl | H | $CH_3$ | Allyl | H | H | — | Br | Harz |
| 29 | 4-Acetyl | H | H | $CH_3$ | Allyl | H | H | — | Br | Harz |
| 30 | 2-Cl | 4-Cl | H | $CH_3$ | $CH_3$ | H | H | — | Br | Harz |
| 31 | 2-Cl | 4-Cl | H | $CH_3$ | Crotyl | H | H | — | Br | Harz |
| 32 | 4-t-Bu | H | H | $CH_3$ | Allyl | H | H | — | Br | 125 |
| 33 | 4-Cl | H | H | H | Allyl | H | H | — | Br | 89 |
| 34 | 4-Cl | H | H | $CH_3$ | Allyl | H | H | $(CH_2)_2$ | Br | 77 |
| 35 | 4-Cl | H | H | $CH_3$ | Allyl | H | H | $CH_2$ | Br | Harz |
| 36 | 2-Cl | 4-Cl | 6-Cl | H | Benzyl | H | H | — | Br | 156 |
| 37 | 4-t-Bu | H | H | $CH_3$ | 4-Br-Benzyl | $CH_3$<br>cis-Dimethyl-morpholin | $CH_3$ | O | Br | 182 |
| 38 | 2-Cl | 4-Cl | H | $CH_3$ | $CH_3$ | H | H | $(CH_2)_2$ | Br | 178 |
| 39 | 2-Cl | 4-Cl | H | $CH_3$ | $C_2H_5$ | H | H | $(CH_2)_2$ | Br | 178 |
| 40 | 2-Cl | 4-Cl | H | $CH_3$ | Allyl | H | H | $(CH_2)_2$ | Br | 180 |
| 41 | 4-t-Bu | H | H | $CH_3$ | 3-Cl-Benzyl | $CH_3$<br>cis-Dimethyl-morpholin | $CH_3$ | O | Br | 192 |
| 42 | 4-Cl | H | H | n-Butyl | Allyl | H | H | — | Br | Harz |
| 43 | 4-Br | H | H | $CH_3$ | Allyl | H | H | — | Br | Harz |
| 44 | 2-Cl | 4-Cl | H | $OCH_3$ | Allyl | H | H | — | Br | Harz |
| 45 | H | H | H | $CH_3$ | Allyl | H | H | — | Br | Harz |
| 46 | 4-t-Bu | H | H | $CH_3$ | 4-Cl-Benzyl | H | H | $(CH_2)_2$ | Br | 169 |
| 47 | 4-t-Bu | H | H | $CH_3$ | 4-Cl-Benzyl | H | H | $CH_2$ | Br | 117 |
| 48 | 3-Cl | 4-Cl | H | $CH_3$ | Allyl | H | H | — | Br | Harz |
| 49 | 4-$CH_3$ | H | H | $CH_3$ | Allyl | H | H | — | Br | Harz |
| 50 | 2-Cl | H | H | $CH_3$ | Allyl | H | H | — | Br | 105 |
| 51 | 3-$CH_3$ | H | H | $CH_3$ | Allyl | H | H | — | Br | 72 |
| 52 | 2-$CH_3$ | H | H | $CH_3$ | Allyl | H | H | — | Br | Harz |
| 53 | 2-Cl | 4-Cl | H | $(CH_3)_2$ | Allyl | H | H | — | Br | 138 |
| 54 | 3-$CH_3$ | 4-Cl | H | $CH_3$ | Allyl | H | H | — | Br | Harz |
| 55 | 4-Cl | H | H | $CH_3$ | Allyl | H | H | — | Br | 147 |
| 56 | 2-Cl | 4-Cl | H | $CH_3$ | Allyl | H | H | — | Br | 174 |
| 57 | 2-Cl | 4-Cl | H | Crotyl<br>$CH_3$<br>Crotyl | Crotyl | H | H | — | Br | 128 |
| 58 | 2-Cl | 4-Cl | H | n-Propyl | Allyl | H | H | $CH_2$ | Br | Harz |
| 59 | 1,1-Dimeth-ylpentyl | 2-Cl | 6-Cl | $CH_3$ | Allyl | H | H | — | Br | 127 |
| 60 | 2-Cl | 3-Cl | 4-Cl | n-Butyl | Allyl | H | H | — | Br | Harz |
| 61 | 1,1-Dimeth-ylpentyl | 2-Br | H | $CH_3$ | $CH_3$ | $CH_3$<br>cis-Dimethyl-morpholin | $CH_3$ | O | Br | Harz |
| 62 | 1,1-Dimeth-ylpentyl | H | H | $CH_3$ | $CH_3$ | $CH_3$<br>cis-Dimethyl-morpholin | $CH_3$ | O | Br | 149 |
| 63 | 1,1-Dimeth-ylpentyl | 2-Cl | H | $CH_3$ | $CH_3$ | $CH_3$<br>cis-Dimethyl-morpholin | $CH_3$ | O | Br | Harz |
| 64 | 1,1-Dimeth-ylbutyl | 2-Cl | H | $CH_3$ | $CH_3$ | $CH_3$<br>cis-Dimethyl-morpholin | $CH_3$ | O | Br | Harz |

| Nr. | $R^1$ | $R^2$ | $R^3$ | $(R^4)_m$ | $R^5$ | $R^6$ | $R^7$ | $(X)_n$ | Y | Fp. (°C) |
|---|---|---|---|---|---|---|---|---|---|---|
| 65 | 4-[1-4-t-Bu-Phenyl)prop-2-yl] | H | H | $CH_3$ | $CH_3$ | $CH_3$ cis-Dimethyl- | $CH_3$ morpholin | O | Br | 210 |

| Nr. | $R^1$ | $R^2$ | $R^3$ | $(R^4)_m$ | $R^5$ | $R^6$ | $R^7$ | $(X)_n$ | Y | Fp. (°C) |
|---|---|---|---|---|---|---|---|---|---|---|
| 66 | 4-(1,1-Diethyl)ethyl | H | H | $CH_3$ | $CH_3$ | $CH_3$ cis-Dimethyl- | $CH_3$ morpholin | O | Br | 198 |
| 67 | 4-Cyclohexyl | 2-Cl | 6-Cl | $CH_3$ | $CH_3$ | H | H | — | Br | 159 |
| 68 | 4-t-Bu | 2-Cl | 6-Cl | $CH_3$ | $CH_3$ | H | H | $CH_2$ | Br | Harz |
| 69 | 4-t-Amyl | 2-Cl | 6-Cl | $CH_3$ | $CH_3$ | H | H | — | Br | 189 |
| 70 | 1,1-Dimethylpentyl | 2-Cl | 6-Cl | $CH_3$ | 4-Cl-Benzyl | H | H | — | Br | 190 |
| 71 | 1,1-Dimethylbutyl | 2-Cl | H | $CH_3$ | Allyl | H | H | — | Br | Harz |
| 72 | 1,1-Dimethylpentyl | 2-Cl | 6-Cl | $CH_3$ | 4-Cl-Benzyl | H | H | $CH_2$ | Br | |
| 73 | 1,1-Dimethylbutyl | 2-Cl | H | $CH_3$ | $CH_3$ | H | H | — | Br | 138 |
| 74 | 4-$CH_3$ | 2-Cl | 6-Cl | $CH_3$ | $CH_3$ | H | H | $CH_2$ | Br | 170 |
| 75 | 1,1-Dimethylpentyl | 2-Cl | 6-Cl | $CH_3$ | $CH_3$ | H | H | $CH_2$ | Br | 180 |
| 76 | 1,1-Dimethylpentyl | 2-Cl | 6-Cl | $CH_3$ | Propargyl | H | H | $CH_2$ | Br | 70 |
| 77 | 1,1-Dimethylpentyl | 2-Cl | 6-Cl | $CH_3$ | $CH_3$ | $CH_3$ | $CH_3$ | $CH_2$ | Br | 120 |
| 78 | 1,1-Dimethylpentyl | 2-Br | H | H | $CH_3$ | H | H | $CH_2$ | Br | 178 |
| 79 | 1,1-Dimethylpentyl | 2-Cl | 6-Cl | $CH_3$ | $CH_3$ | H | H | — | Br | 123 |
| 80 | 1,1-Dimethylpentyl | 2-Cl | 6-Cl | $CH_3$ | $CH_3$ | H | H | $(CH_2)_2$ | Br | 138 |
| 81 | 1,1-Dimethylpentyl | 2-Cl | 6-Cl | $CH_3$ | Crotyl | H | H | $CH_2$ | Br | 136 |
| 82 | 1,1-Dimethylpentyl | 2-Cl | 6-Cl | $CH_3$ | Propargyl | H | H | — | Br | 71 |
| 83 | 1,1-Dimethylpentyl | 2-Cl | 6-Cl | $CH_3$ | Crotyl | H | H | — | Br | 107 |
| 84 | 1,1-Dimethylpentyl | 2-Cl | 6-Cl | $CH_3$ | $CH_3$ | H | H | — | J | Harz |
| 85 | 1,1-Dimethylpentyl | 2-Cl | 6-Cl | $CH_3$ | Allyl | H | H | $CH_2$ | Br | 131 |
| 86 | 1,1-Dimethylpentyl | 2-Cl | 6-Cl | $CH_3$ | 4-$CF_3$-Benzyl | H | H | $CH_2$ | Br | 204 |
| 87 | 4-$CH_3$ | 2-Cl | 6-Cl | $CH_3$ | Allyl | H | H | $CH_2$ | Br | 145 |
| 88 | 3-$CH_3$ | H | H | $CH_3$ | Propargyl | H | H | — | Br | 110 |
| 89 | 4-t-Bu | 2-$CH_3$ | H | $CH_3$ | Allyl | H | H | — | Br | Harz |
| 90 | $C_6H_5$ | H | H | $CH_3$ | $CH_3$ | $CH_3$ | $CH_3$ | O | Br | |
| 91 | 4-(2-Chlor-1,1-dimethyl-ethyl) | H | H | $CH_3$ | $CH_3$ | $CH_3$ cis-Dimethyl- | $CH_3$ morpholin | O | Br | Harz |
| 92 | 4-$CF_3$ | H | H | $CH_3$ | Allyl | H | H | — | Br | Harz |
| 93 | 2-$CF_3$ | H | H | $CH_3$ | Allyl | H | H | — | Br | 118 |
| 94 | 3-$CF_3$ | H | H | $CH_3$ | Allyl | H | H | — | Br | 124 |
| 95 | 2-Cl | 4-Cl | 6-Cl | H | Allyl | H | H | — | Br | Harz |
| 96 | 4-t-Bu | 2-$CH_3$ | H | $CH_3$ | Benzyl | H | H | — | Br | 125 |
| 97 | 4-(1,1-Diethyl)ethyl | H | H | $CH_3$ | 4-Cl-Benzyl | H | OH | $CH_2$ | Cl | 92 |

Die Wirkstoffe zeigen eine starke Wirksamkeit gegen Mikroorganismen. Sie dienen insbesondere zur Verhütung und Heilung von Pflanzenkrankheiten, die durch Pilze verursacht werden, wie z. B. *Botrytis cinerea* an Reben und Erdbeeren, *Monilia fructigena* an Äpfeln und Birnen, *Phytophthora infestans* an Kartoffeln und Tomaten, *Plasmopara viticola* an Reben, *Alternaria solani* an Tomaten, *Erysiphe graminis* (echter Mehltau) an Getreide und *Erysiphe cichoracearum* (echter Mehltau) an Kürbisgewächsen. Ferner zeigen sie eine gute Wirksamkeit gegen holzverfärbende und holzzerstörende Pilze wie *Chaetomium globosum, Pullularia pullulans, Sclerophoma pityophyla, Aspergillus niger, Coniophora puteana* und *Polystictus versicolor.* Ausserdem besitzen die Wirkstoffe eine vorteilhafte bakterizide Wirksamkeit. Sie eignen sich zur Bekämpfung von Mikroorganismen (Mikroben) in Rückkühlwerken, in der Papierindustrie oder in Luftbefeuchtungsanlagen. Ferner können sie als Desinfektionsmittel oder Konservierungsmittel für den technischen Bereich sowie als Bakterizide im Pflanzenschutz eingesetzt werden.

Folgende Mikroorganismen lassen sich beispielsweise mit den Wirkstoffen bekämpfen:

*Staphylococcus aureus, Escherichia coli, Klebsiella pneumoniae, Citrobacter freundii, Proteus vulgaris, Pseudomonas aeriginosa, Pseudomonas fluorescens, Xanthomonas vesicatoria, Xanthomonas malvacearum, Erwinia carotovora, Erwinia amylovora, Desulfovibrio desulfuricans, Streptoverticillium rubtrireticuli, Aspergillus niger, Aspergillus versicolor, Penicillium funiculosum, Paecilomyces variotii, Trichoderma viride, Chaetomium globosum, Candida albicans, Geotrichum candidans, Monilia sitophila.*

Die Verbindungen sind in Wasser leicht löslich. Sie werden daher bevorzugt in Form ihrer wässerigen Lösung angewendet. Konzentrierte Zubereitungen können auch mit organischen Lösungsmitteln, z. B. Ethanol, hergestellt werden.

Die üblichen Anwendungskonzentrationen betragen 0,01 bis 1% Wirkstoff, bezogen auf das Gewicht des zu schützenden Materials; beim Einsatz zur Wasserbehandlung bei der Erdölförderung, in Schwimmbädern, Rückkühlwerken, Luftbefeuchtungsanlagen, Blumenfrischhaltemittel oder in der Papierindustrie sind Wirkstoffmengen von 5 bis 100 ppm ausreichend, um eine Mikroorganismenentwicklung zu unterdrücken. Gebrauchsfertige Desinfektionsmittellösungen enthalten 0,5 bis 10% (Gew.-%) Wirkstoff.

Einige der Wirkstoffe können auch zur Bekämpfung humanpathogener Pilze eingesetzt werden, wie z. B. gegen *Trichophyton mentagrophytes* oder *Candida albicans.*

Die fungiziden bzw. bakteriziden Mittel enthalten 0,1 bis 95% (Gew.-%) Wirkstoff, vorzugsweise 0,5 bis 90%. Die Aufwandmengen liegen je nach Art des gewünschten Effektes zwischen 0,1 und 5 kg Wirkstoff/ha.

Die Wirkstoffe können auch zusammen mit anderen Wirkstoffen, z. B. Herbiziden, Insektiziden, Wachstumsregulatoren und anderen Fungiziden, oder auch mit Düngemitteln vermischt und ausgebracht werden. In vielen Fällen erhält man bei der Mischung mit Fungiziden auch eine Vergrösserung des fungiziden Wirkungsspektrums; bei einer Anzahl dieser Fungizidmischungen treten auch synergistische Effekte auf, d. h. die fungizide Wirksamkeit des Kombinationsproduktes ist grösser als die der addierten Wirksamkeiten der Einzelkomponenten. Eine besonders günstige Vergrösserung des Wirkungsspektrums wird mit folgenden Fungiziden erzielt:

Manganethylenbisdithiocarbamat
Mangenzinkethylenbisdithiocarbamat
Ammoniakkomplex von Zink-(N,N-ethylenbisdithiocarbamat)
N-Trichlormethylthiotetrahydrophtalimid
N-Trichlormethylphthalimid
5-Ethoxy-3-trichlormethyl-1,2,3-thiadiazol
2-Methoxycarbonylaminobenzimidazol
2-Rhodanmethylthiobenzthiazol
1,4-Dichlor-2,5-dimethoxybenzol
2,3-Dichlor-6-methyl-1,4-oxathiin-5-carbonsäureanilid
2-Methyl-5,6-dihydro-4H-pyran-3-carbonsäureanilid
2,4,5-Trimethylfuran-3-carbonsäureanilid
2-Methylfuran-3-carbonsäureanilid
2,5-Dimethylfuran-3-carbonsäurecyclohexylamid
N-Cyclohexyl-N-methoxy-2,5-dimethylfuran-3-carbonsäureamid
5-Methyl-5-vinyl-3-(3,5-dichlorphenyl)-2,4-dioxo-1,3-oxazolidin
3-(3,5-Dichlorphenyl)-5-methyl-5-methoxymethyl-1,3-oxazolidin-2,4-dion

Die Verbindungen können aber auch mit folgenden Fungiziden kombiniert werden:

Dithiocarbamate und deren Derivate, wie
Ferridimethyldithiocarbamat
Zinkdimethyldithiocarbamat
Zinkethylenbisdithiocarbamat
Tetramethylthiuramdisulfide
Zink-(N,N-propylenbisdithiocarbamat)
Ammoniakkomplex von Zink-(N,N'-propylenbisdithiocarbamat), und
N,N'-Polypropylenbis(thiocarbamoyl)disulfid-
nitroderivate, wie
Dinitro-(1-methylheptyl)phenylcrotonat
2-sek.-Butyl-4,6-dinitrophenyl-3,3-dimethylacrylat
2-sek.-Butyl-4,6-dinitrophenylisopropylcarbonat
heterocyclische Strukturen, wie
2-Heptadecyl-2-imidazolinacetat
2,4-Dichlor-6-(o-chloranilino)-s-triazin
O,O-Diethylphthalimidophonothioat
5-Amino-1-[bis(dimethylamino)phosphinyl]-3-phenyl-1,2,4-triazol
2,3-Dicyano-1,4-dithiaanthrachinon
2-Thio-1,3-dithio-(4,5,6)-chinoxalin
1-(Butylcarbamoyl)-2-benzimidazolcarbaminsäuremethylester
4-(2-Chlorphenylhydrazono)-3-methyl-5-isoxazolon
Pyridin-2-thio-1-oxid

8-Hydroxychinolin bzw. dessen Kupfersalz
2,3-Dihydro-5-carboxanilido-6-methyl-1,4-oxathiin-4,4-dioxid
2,3-Dihydro-5-carboxanilido-6-methyl-1,4-oxathiin
2-[Furyl-(2)]-benzimidazol
Piperazin-1,4-diylbis-1-(2,2,2-trichlorethylformamid
2-[Thiazolyl-(4)]-benzimidazol
5-Butyl-2-dimethylamino-4-hydroxy-6-methylpyrimidin
Bis-(p-chlorphenyl)-3-pyridinmethanol
1,2-Bis-(3-methoxycarbonyl-2-thioureido)-benzol sowie

verschiedene Fungizide, wie
Dodecylguanidinacetat
3-[3-(3,5-Dimethyl-2-oxycyclohexyl)-2-hydroxyethyl]glutarimid
Hexachlorbenzol
N-Dichlorfluormethylthio-N',N'-dimethyl-N-phenylschwefelsäurediamid
2,5-Dimethylfuran-3-carbonsäureanilid
2-Methylbenzoesäureanilid
2-Jodbenzoesäureanilid
1-(3,4-Dichloranilino)-1-formylamino-2,2,2-trichlorethan
2,6-Dimethyl-N-tridecylmorpholin bzw. dessen Salze
2,6-Dimethyl-N-cyclododecylmorpholin bzw. dessen Salze
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
(2-Chlorphenyl)-α-(4-chlorphenyl)-5-pyrimidinmethanol

Die Wirkstoffe werden beispielsweise in Form von direkt versprühbaren Lösungen, Pulvern, Suspensionen, auch hochprozentige wässerige, ölige oder sonstige Suspensionen oder Dispersionen, Emulsionen, Öldispersionen, Pasten, Stäubemitteln, Streumitteln, Granulaten durch Versprühen, Vernebeln, Verstäuben, Verstreuen oder Giessen ausgebracht. Die Anwendungsformen richten sich ganz nach den Verwendungszwecken; sie sollten in jedem Fall möglichst die feinste Verteilung der neuen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, z. B. Dimethylformamid, Dimethylsulfoxid, N-Methylpyrrolidon, Wasser in Betracht.

Wässerige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutylnaphthalinsulfonsäure, Laurylethersulfate, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. die Naphthalinsulfonsäuren mit Phenol und Formaldehyd, Polyoxyethylenoctylphenolether, ethoxyliertes Isooctylphenol-, Octylphenol-, Nonylphenol-, Alkylphenolpolyglykolether, Tributylphenylpolyglykolether, Alkylarylpolyetheralkohole, Isotridecylalkohol, Fettalkoholethylenoxidkondensate, ethoxyliertes Rizinusöl, Polyoxyathylenalkylether, ethoxyliertes Polyoxypropylen, Laurylalkoholpolyglykoletheracetal, Sorbitester, Lignin, Sulfitablaugen und Methylcellulose in Betracht.

Pulver, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden wie Silicagel, Kieselsäuren, Kieselgele, Silikate, Talkum, Kaolin, Attaclay, Kalkstein, Kreide, Bolus, Löss, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehle, Baumrinden-, Holz- und Nussschalenmehl, Cellulosepulver und andere feste Trägerstoffe.

Beispiele für solche Zubereitungen:

I. Man vermischt 90 Gew.-Teile der Verbindung 9 mit 10 Gew.-Teilen N-Methyl-α-pyrrolidon und erhält eine Lösung, die zur Anwendung in Form kleinster Tropfen geeignet ist.

II. 20 Gew.-Teile der Verbindung 10 werden in einer Mischung gelöst, die aus 89 Gew.-Teilen Xylol, 10 Gew.-Teilen des Anlagerungsproduktes von 8 bis 10 mol Ethylenoxid an 1 mol Ölsäure-N-monodecylbenzolsulfonsäure und 5 Gew.-Teilen des Anlagerungsproduktes von 50 mol Ethylenoxid an 1 mol Rizinusöl besteht. Durch Ausgiessen und feines Verteilen der Lösung in Wasser erhält man eine wässerige Dispersion.

III. 20 Gew.-Teile der Verbindung Nr. 12 werden in einer Mischung gelöst, die aus 40 Gew.-Teilen Cyclohexanon, 30 Gew.-Teilen Isobutanol, 20 Gew.-Teilen des Anlagerungsproduktes von 40 mol Ethylenoxid an 1 mol Rizinusöl besteht. Durch Eingiessen und feines Verteilen der Lösung in Wasser erhält man eine wässerige Dispersion.

IV. 20 Gew.-Teile der Verbindung Nr. 21 werden in einer Mischung gelöst, die aus 25 Gew.-Teilen Cyclohexanol, 65 Gew.-Teilen einer Mineralölfraktion vom Sdp. 210 bis 280° C und 10 Gew.-Teilen des Anlagerungsproduktes von 40 mol Ethylenoxid an 1 mol Rizinusöl besteht. Durch Eingiessen und feines Verteilen der Lösung in Wasser, erhält man eine wässerige Dispersion.

V. 80 Gew.-Teile der Verbindung Nr. 23 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 5 Gew.-Teile der Verbindung Nr. 25 werden mit 95 Gew.-Teilen feinteiligem Kaolin innig vermischt. Man erhält auf diese Weise ein Stäubemittel, das 5 Gew.-% des Wirkstoffs enthält.

VII. 30 Gew.-Teile der Verbindung Nr. 28 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 30 werden mit 30 Teilen Natriumsalz eines Phenolsulfonsäureharnstofformaldehydkondensates, 2 Teilen Kieselgel und 48 Teilen Wasser innig vermischt. Man erhält eine stabile wässerige Dispersion.

IX. 20 Teile der Verbindung Nr. 31 werden mit 2 Teilen Calciumsalz der Dodecylbenzolsulfonsäure, 8 Teilen Fettalkoholpolyglykolether, 2 Teilen Natriumsalz eines Phenolsulfonsäureharnstofformaldehydkondensates und 68 Teilen eines paraffinischen Mineralöls innig vermischt. Man erhält eine stabile ölige Dispersion.

Die folgenden Versuche belegen die biologische Wirkung der neuen Verbindungen. Vergleichsmittel ist der bekannte Wirkstoff N-Trichlormethylthiotetrahydrophthalimid (A).

*Versuch 1:*

Wirksamkeit gegen *Botrytis cinerea* an Paprika

Paprikasämlinge der Sorte Neusiedler Ideal Elite werden, nachdem sich 4 bis 5 Blätter gut entwickelt haben, mit wässerigen Suspensionen, die 80% Wirkstoff und 20% Emulgiermittel in der Trockensubstanz enthalten, tropfnass gespritzt. Nach dem Antrocknen des Spritzbelages werden die Pflanzen mit einer Konidienaufschwemmung des Pilzes *Botrytis cinerea* besprüht und bei 22 bis 24° C in eine Kammer mit hoher Luftfeuchtigkeit gestellt. Nach 5 d hat sich die Krankheit auf den unbehandelten Kontrollpflanzen so stark entwickelt, dass die entstandenen Blattnekrosen den überwiegenden Teil der Blätter bedecken.

Das Ergebnis des Versuches zeigt, dass beispielsweise die Wirkstoffe 59, 63, 69, 70, 72, 75, 76, 77, 78, 79, 80, 81, 82, 83 und 84 bei Anwendung als 0,05%ige Wirkstoffbrühe eine bessere fungizide Wirkung (beispielsweise 97%) zeigen als der bekannte Wirkstoff A (beispielsweise 70%).

*Versuch 2:*

Wirksamkeit gegen *Phytophthora infestans* an Tomaten

Blätter von Topfpflanzen der Sorte Grosse Fleischtomate werden mit wässeriger Spritzbrühe, die 80% Wirkstoff und 20% Emulgiermittel in der Trockensubstanz enthält, besprüht. Nach dem Antrocknen des Spritzbelages werden die Blätter mit einer Zoosporenaufschwemmung des Pilzes *Phytophthora infestans* infiziert. Die Pflanzen werden dann in einer wasserdampfgesättigten Kammer bei Temperaturen zwischen 16 unf 18° C aufgestellt. Nach 5 d hat sich die Krankheit auf den unbehandelten, jedoch infizierten Kontrollpflanzen so stark entwickelt, dass die fungizide Wirksamkeit der Substanzen beurteilt werden kann.

Das Ergebnis des Versuches zeigt, das beispielsweise die Wirkstoffe 59, 72, 75, 80, 81, 83 und 84 bei der Anwendung als 0,025%ige Wirkstoffbrühe eine bessere fungizide Wirkung (beispielsweise 97%) zeigen als der bekannte Wirkstoff A (beispielsweise 60%).

*Versuch 3:*

Wirksamkeit gegen Weizenmehltau

Blätter von in Töpfen gewachsenen Weizenkeimlingen der Sorte Jubilar werden mit wässeriger Spritzbrühe, die 80% Wirkstoff und 20% Emulgiermittel in der Trockensubstanz enthält, besprüht und 24 h nach dem Antrocknen des Spritzbelages mit Oidien (Sporen) des Weizenmehltaus (*Erysiphe graminis* var. *tritici*) bestäubt. Die Versuchspflanzen werden anschliessend im Gewächshaus bei Temperaturen zwischen 20 und 22° C und 75 bis 80% relativer Luftfeuchte aufgestellt. Nach 7 d wird das Ausmass der Mehltauentwicklung ermittelt.

Das Ergebnis des Versuches zeigt, dass beispielsweise die Wirkstoffe 9, 10, 12, 21, 23, 25, 28, 30, 31, 32, 34, 37, 38, 39, 41, 43, 46, 47, 53, 71, 73 bei der Anwendung als 0,025%ige Wirkstoffbrühe eine sehr gute fungizide Wirkung (beispielsweise 100%) zeigen.

**Patentansprüche** für die Vertragsstaaten BE, CH, DE, FR, GB, IT, LI, NL, SE

1. Phenylpropylammoniumsalz der Formel (I)

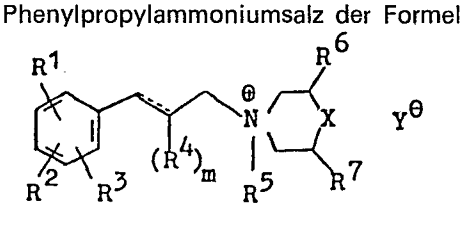

(I)

in der $R^1$, $R^2$, $R^3$ jeweils unabhängig voneinander Wasserstoff, $C_1$-$C_8$-Alkyl, halogensubstituiertes $C_1$-$C_4$-Alkyl, ggf. durch Halogen oder $C_1$-$C_4$-Alkyl substituiertes Phenyl, ggf. durch Halogen substituiertes Benzyl, 2,4,6-Trimethylbenzyl, $C_3$-$C_7$-Cycloalkyl, $C_1$-$C_4$-Alkoxy, $C_2$-$C_4$-Acyl oder Halogen, $R^4$ Wasserstoff, $C_1$-$C_6$-Alkyl, $C_3$-$C_4$-Alkenyl, $C_1$-$C_3$-Alkoxy, $R^5$ $C_1$-$C_4$-Alkyl, $C_4$-Alkenyl, Propargyl oder ggf. durch Halogen oder $C_1$-$C_4$-Alkyl substituiertes Benzyl, $R^6$ und $R^7$ unabgängig voneinander Wasserstoff, Methyl, Ethyl, Propyl, $CH_2OH$, OH und X $CH_2$, O, S, C=O oder

| |
$CH_2CH-R^8$, bedeutet,

wobei $R^8$ Alkyl bedeutet und m 1 oder 2, und $Y^{\ominus}$ das Anion einer nichtphytotoxischen Säure bedeutet,

ausser N-n-Butyl-3-(3-ethoxyphenyl)propyl-piperidiniumoxalat.

2. Phenylpropylammoniumsalz gemäss Anspruch 1 der Formel

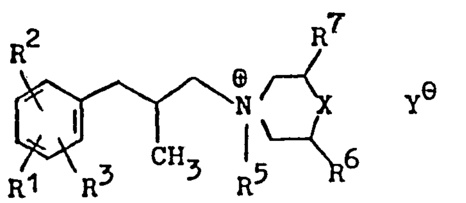

in der $R^1$ $C_1$-$C_8$-Alkyl oder ggf. durch Halogen substituiertes Benzyl, $R^2$ und $R^3$ unabhängig voneinander Wasserstoff, Brom oder Chlor, $R^5$ $C_1$-$C_4$-Alkyl, $C_4$-Alkenyl, Propargyl oder ggf. durch Halogen oder $C_1$-$C_4$-Alkyl substituiertes Benzyl, $R^6$ und $R^7$ unabhängig voneinander Wasserstoff oder Methyl, X O, $CH_2$, und $Y^{\ominus}$ das Anion einer nichtphytotoxischen Säure bedeutet.

3. Fungizides Mittel, enthaltend ein Salz gemäss Anspruch 1.

4. Fungizides Mittel, enthaltend einen inerten Trägerstoff und ein Salz gemäss Anspruch 1.

5. Fungizides Mittel, enthaltend ein Salz gemäss Anspruch 2.

6. Verfahren zur Bekämpfung von Pilzen, dadurch gekennzeichnet, dass man ein Phenylpropylammoniumsalz der Formel (I')

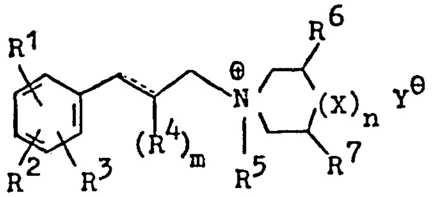

(I')

in der $R^1$, $R^2$, $R^3$ jeweils unabhängig voneinander Wasserstoff, $C_1$-$C_8$-Alkyl, halogensubstituiertes $C_1$-$C_4$-Alkyl, ggf. durch Halogen oder $C_1$-$C_4$-Alkyl substituiertes Phenyl, ggf. durch Halogen substituiertes Benzyl, 2,4,6-Trimethylbenzyl, $C_3$-$C_7$-Cycloalkyl, $C_1$-$C_4$-Alkoxy, $C_2$-$C_4$-Acyl oder Halogen, $R^4$ Wasserstoff, $C_1$-$C_6$-Alkyl, $C_3$-$C_4$-Alkenyl, $C_1$-$C_3$-Alkoxy, $R^5$ $C_1$-$C_4$-Alkyl, $C_2$-$C_4$-Alkenyl, Propargyl oder ggf. durch Halogen oder $C_1$-$C_4$-Alkyl substituiertes Benzyl, $R^6$ und $R^7$ unabhängig voneinander Wasserstoff, Methyl, Ethyl, Propyl, $CH_2OH$, OH und X $CH_2$, O, S, C=O, $(CH_2)_2$ oder $CH_2CH-R^8$ bedeutet,

wobei $R^8$ Alkyl bedeutet und m 1, 2, n 0 und 1 und $Y^{\ominus}$ das Anion einer nichtphytotoxischen Säure bedeutet,

auf Pilze oder die vor Pilzbefall zu schützenden Gegenstände einwirken lässt.

7. Verfahren zur Herstellung von Phenylpropylammoniumsalzen gemäss Anspruch 1, dadurch gekennzeichnet, dass man ein Amin der Formel

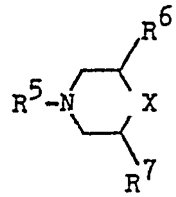

in der $R^5$, $R^6$, $R^7$ und X die im Anspruch 1 genannten Bedeutungen haben, ggf. in Gegenwart eines Verdünnungsmittels umsetzt mit einer Verbindung der Formel

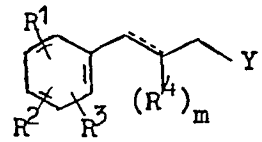

in der $R^1$, $R^2$, $R^3$, $R^4$ und m die im Anspruch 1 genannten Bedeutungen haben und Y Cl, Br oder J bedeutet.

**Patentansprüche** für den Vertragsstaat: AT

1. Fungizides Mittel, enthaltend ein Phenylpropylammoniumsalz der Formel (I)

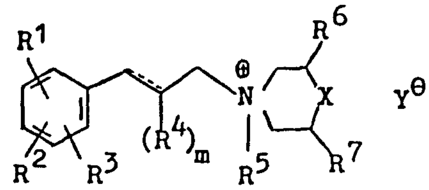

(I)

in der $R^1$, $R^2$, $R^3$ jeweils unabhängig voneinander Wasserstoff, $C_1$-$C_8$-Alkyl, halogensubstituiertes $C_1$-$C_4$-Alkyl, ggf. durch Halogen oder $C_1$-$C_4$-Alkyl substituiertes Phenyl, ggf. durch Halogen substituiertes Benzyl, 2,4,6-Trimethylbenzyl, $C_3$-$C_7$-Cycloalkyl, $C_1$-$C_4$-Alkoxy, $C_2$-$C_4$-Acyl oder Halogen, $R^4$ Wasserstoff, $C_1$-$C_6$-Alkyl, $C_3$-$C_4$-Alkenyl, $C_1$-$C_3$-Alkoxy, $R^5$ $C_1$-$C_4$-Alkyl, $C_4$-Alkenyl, Propargyl oder ggf. durch Halogen oder $C_1$-$C_4$-Alkyl substituiertes Benzyl, $R^6$ und $R^7$ unabhängig voneinander Wasserstoff, Methyl, Ethyl, Propyl, $CH_2OH$, OH und X $CH_2$, O, S, C=O oder

| |
$CH_2CH-R^8$, bedeutet,

wobei $R^8$ Alkyl bedeutet und m 1 oder 2, und $Y^{\ominus}$ das Anion einer nichtphytotoxischen Säure bedeutet,

ausser N-n-Butyl-3-(3-ethoxyphenyl)propyl-piperidiniumoxalat.

2. Fungizides Mittel gemäss Anspruch 1, enthaltend ein Phenylpropylammoniumsalz der Formel

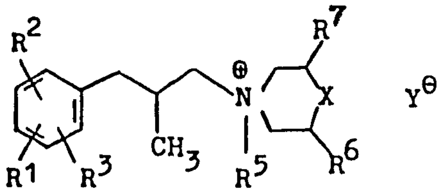

in der $R^1$ $C_1$-$C_8$-Alkyl oder ggf. durch Halogen substituiertes Benzyl, $R^2$ und $R^3$ unabhängig voneinander Wasserstoff, Brom oder Chlor, $R^5$ $C_1$-$C_4$-Alkyl, $C_4$-Alkenyl, Propargyl oder ggf. durch Halogen oder $C_1$-$C_4$-Alkyl substituiertes Benzyl, $R^6$ und $R^7$ unabhängig voneinander Wasserstoff oder Methyl, X O oder $CH_2$ und $Y^{\ominus}$das Anion einer nichtphytotoxischen Säure bedeutet.

3. Fungizides Mittel, enthaltend einen inerten Trägerstoff und ein Salz gemäss Anspruch 1.

4. Fungizides Mittel, enthaltend einen inerten Trägerstoff und ein Salz gemäss Anspruch 2.

5. Verfahren zur Bekämpfung von Pilzen, dadurch gekennzeichnet, dass man ein Phenylpropylammoniumsalz der Formel (I')

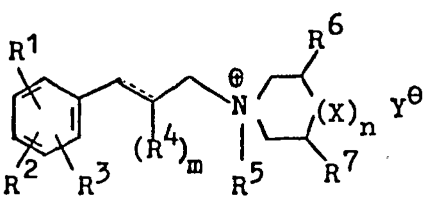

(I')

in der $R^1$, $R^2$, $R^3$ jeweils unabhängig voneinander Wasserstoff, $C_1$-$C_8$-Alkyl, halogensubstituiertes $C_1$-$C_4$-Alkyl, ggf. durch Halogen oder $C_1$-$C_4$-Alkyl substituiertes Phenyl, ggf. durch Halogen substituiertes Benzyl, 2,4,6-Trimethybenzyl, $C_3$-$C_7$-Cycloalkyl, $C_1$-$C_4$-Alkoxy, $C_2$-$C_4$-Acyl oder Halogen, $R^4$ Wasserstoff, $C_1$-$C_6$-Alkyl, $C_3$-$C_4$-Alkenyl, $C_1$-$C_3$-Alkoxy, $R^5$ $C_1$-$C_4$-Alkyl, $C_2$-$C_4$-Alkenyl, Propargyl oder ggf. durch Halogen oder $C_1$-$C_4$-Alkyl substituiertes Benzyl, $R^6$ und $R^7$ unabhängig voneinander Wasserstoff, Methyl, Ethyl, Propyl, $CH_2OH$, OH und X $CH_2$, O, S, C=O $(CH_2)_2$ oder $CH_2CH-R^8$ bedeutet,
wobei $R^8$ Alkyl bedeutet und m 1, 2, n 0 und 1 und $Y^{\ominus}$ das Anion einer nichtphytotoxischen Säure bedeutet,
auf Pilze oder die vor Pilzbefall zu schützenden Gegenstände einwirken lässt.

6. Verfahren zur Herstellung von Phenylpropylammoniumsalzen wie im Anspruch 1 definiert, dadurch gekennzeichnet, dass man ein Amin der Formel

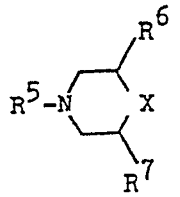

in der $R^5$, $R^6$, $R^7$ und X die im Anspruch 1 genannten Bedeutungen haben, ggf. in Gegenwart eines Verdünnungsmittels umsetzt mit einer Verbindung der Formel

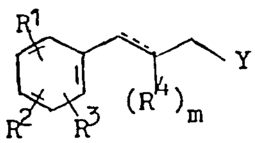

in der $R^1$, $R^2$, $R^3$, $R^4$ und m die im Anspruch 1 genannten Bedeutungen haben und Y Cl, Br oder J bedeutet.

**Revendications** pour les Etats contractants BE, CH, DE, FR, GB, IT, LI, NL, SE

1. Sel de phénylpropylammonium de la formule (I):

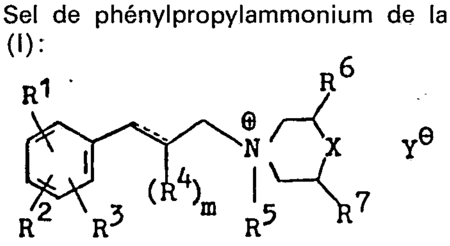

(I)

dans laquelle:

$R^1$, $R^2$ et $R^3$ désignent chacun, indépendamment l'un de l'autre, un atome d'hydrogène, un radical alkyle en $C_1$ à $C_8$, un radical alkyle en $C_1$ à $C_4$ halosubstitué, un groupe phényle éventuellement halo- ou alkyl(en $C_1$ à $C_4$)-substitué, benzyle éventuellement halosubstitué, triméthyl-2,4,6 benzyle, cycloalkyle en $C_3$ à $C_7$, alcoxy en $C_1$ à $C_4$, acyle en $C_2$ à $C_4$ ou halogène,

$R_4$ désigne un atome d'hygrogène, un radical alkyle en $C_1$ à $C_6$, alcényle en $C_3$ ou $C_4$ ou alcoxy en $C_1$ à $C_3$,

$R^5$ désigne un radical alkyle en $C_1$ à $C_4$ ou alcényle en $C_4$, un groupe propargyle ou un groupe benzyle éventuellement halo- ou alkyl(en $C_1$ à $C_4$)-substitué,

$R^6$ et $R^7$ désignent chacun, indépendamment l'un de l'autre, un atome d'hydrogène, un radical méthyle, éthyle ou propyle, $CH_2OH$ ou OH,

X représente $CH_2$, O, S, C=O ou $\overset{|}{C}H_2\overset{|}{C}H-R^8$, où $R^8$ est un radical alkyle,

m vaut 1 ou 2, et

$Y^{\ominus}$ représente l'anion d'un acide non phytotoxique, à l'exception de l'oxalate de N-n-butyl-3-(éthoxy-3 phényl)propylpipéridinium.

2. Sel de phénylpropylammonium selon la revendication 1, de la formule:

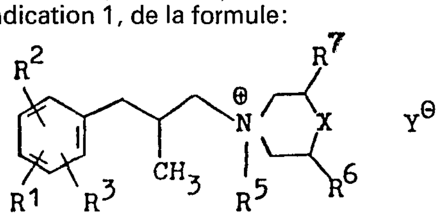

dans laquelle:

$R^1$ désigne un radical alkyle en $C_1$ à $C_8$ ou un groupe benzyle éventuellement halosubstitué,

$R^2$ et $R^3$ désignent chacun, indépendamment l'un de l'autre, un atome d'hydrogène, de brome ou de chlore,

$R^5$ désigne un radical alkyle en $C_1$ à $C_4$ ou alcényle en $C_4$, un groupe propargyle ou un

groupe benzyle éventuellement halo- ou alkyl(en $C_1$ à $C_4$)-substitué,

$R^6$ et $R^7$ désignent, indépendamment l'un de l'autre, un atome d'hydrogène ou un radical méthyle,

X = 0 ou $CH_2$, et

$Y^\ominus$ est l'anion d'un acide non phytotoxique.

3. Composition fongicide, contenant un sel selon la revendication 1.

4. Composition fongicide, contenant un sel selon la revendication 1 et une matière-support inerte.

5. Composition fongicide, contenant un sel selon la revendication 2.

6. Procédé de lutte contre des champignons, caractérisé en ce que l'on fait agir sur les champignons ou sur les objets à protéger des champignons un sel de phénylpropylammonium de la formule (I'):

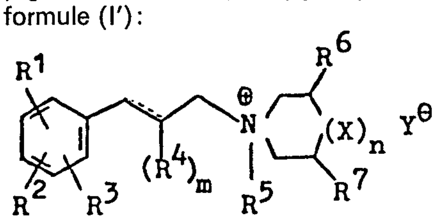
(I')

dans laquelle:

$R^1$, $R^2$ et $R^3$ désignent chacun, indépendamment l'un de l'autre, un atome d'hydrogène, un radical alkyle en $C_1$ à $C_8$, un radical alkyle en $C_1$ à $C_4$ halosubstitué, un groupe phényle éventuellement halo- ou alkyl(en $C_1$ à $C_4$)-substitué, benzyle éventuellement halosubstitué, triméthyl-2,4,6 benzyle, cycloalkyle en $C_3$ à $C_7$, alcoxy en $C_1$ à $C_4$, acyle en $C_2$ à $C_4$ ou halogène,

$R^4$ désigne un atome d'hydrogène, un radical alkyle en $C_1$ à $C_6$, alcényle en $C_3$ ou $C_4$ ou alcoxy en $C_1$ à $C_3$,

$R^5$ désigne un radical alkyle en $C_1$ à $C_4$ ou alcényle en $C_2$ à $C_4$, un groupe propargyle ou un groupe benzyle éventuellement halo- ou alkyl(en $C_1$ à $C_4$)-substitué,

$R^6$ et $R^7$ désignent chacun, indépendamment l'un de l'autre, un atome d'hydrogène, un radical méthyle, éthyle ou propyle, $CH_2OH$ ou OH,

X représente $CH_2$, O, S, C=O, $(CH_2)_2$ ou $CH_2CH-R^8$, où $R^8$ est un radical alkyle,

m vaut 1 ou 2 et n vaut 0 ou 1, et

$Y^\ominus$ représente l'anion d'un acide non phytotoxique.

7. Procédé de préparation de sels de phénylpropylammonium selon la revendication 1, caractérisé en ce que l'on fait réagir une amine de la formule

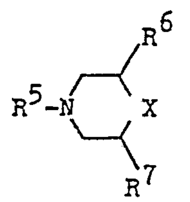

dans laquelle $R^5$, $R^6$, $R^7$ et X possèdent les significations définies dans la revendication 1, éventuellement dans un diluant, avec un composé de la formule:

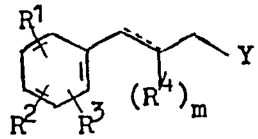

dans laquelle $R^1$, $R^2$, $R^3$, $R^4$ et m possèdent les significations définies dans la revendication 1, et Y représente un atome de chlore, de brome ou d'iode.

**Revendications** pour l'Etat contractant: AT

1. Composition fongicide, contenant un sel de phénylpropylammonium de la formule (I):

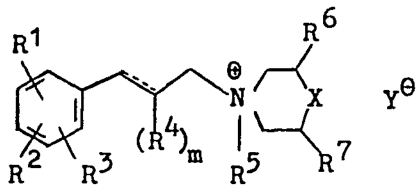
(I)

dans laquelle:

$R^1$, $R^2$ et $R^3$ désignent chacun, indépendamment l'un de l'autre, un atome d'hydrogène, un radical alkyle en $C_1$ à $C_8$, un radical alkyle en $C_1$ à $C_4$ halosubstitué, un groupe phényle éventuellement halo- ou alkyl(en $C_1$ à $C_4$)-substitué, benzyle éventuellement halosubstitué, triméthyl-2,4,6 benzyle, cycloalkyle en $C_3$ à $C_7$, alcoxy en $C_1$ à $C_4$, acyle en $C_2$ à $C_4$ ou halogène,

$R^4$ désigne un atome d'hydrogène, un radical alkyle en $C_1$ à $C_6$, alcényle en $C_3$ ou $C_4$ ou alcoxy en $C_1$ à $C_3$,

$R^5$ désigne un radical alkyle en $C_1$ à $C_4$ ou alcényle en $C_4$, un groupe propargyle ou un groupe benzyle éventuellement halo- ou alkyl(en $C_1$ à $C_4$)-substitué,

$R^6$ et $R^7$ désignent chacun, indépendamment l'un de l'autre, un atome d'hydrogène, un radical méthyle, éthyle ou propyle, $CH_2OH$ ou OH,

X représente $CH_2$, O, S, C=O ou $CH_2CH-R^8$, où $R^8$ est un radical alkyle,

m vaut 1 ou 2, et

$Y^\ominus$ représente l'anion d'un acide non phytotoxique, à l'exception de l'oxalate de N-n-butyl-3-(éthoxy-3 phényl)propylpipéridinium.

2. Composition fongicide selon la revendication 1, contenant un sel de phénylpropylammonium de la formule:

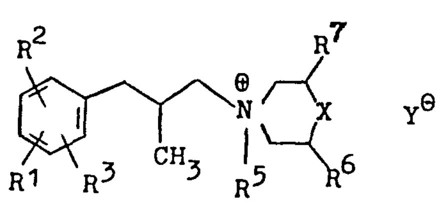

dans laquelle:

$R^1$ désigne un radical alkyle en $C_1$ à $C_8$ ou un groupe benzyle éventuellement halosubstitué,

$R^2$ et $R^3$ désignent chacun, indépendamment l'un de l'autre, un atome d'hydrogène, de brome ou de chlore,

$R^5$ désigne un radical alkyle en $C_1$ à $C_4$ ou alcényle en $C_4$, un groupe propargyle ou un groupe benzyle éventuellement halo- ou alkyl(en $C_1$ à $C_4$)-substitué,

$R^6$ et $R^7$ désignent, indépendamment l'un de l'autre, un atome d'hydrogène ou un radical méthyle,

X = 0 ou $CH_2$, et

$Y^{\ominus}$ est l'anion d'un acide non phytotoxique.

3. Composition fongicide, contenant un sel selon la revendication 1 et une matière-support inerte.

4. Composition fongicide, contenant un sel selon la revendication 2 et une matière-support inerte.

5. Procédé de lutte contre des champignons, caractérisé en ce que l'on fait agir sur les champignons ou sur les objets à protéger des champignons un sel de phénylpropylammonium de la formule (I'):

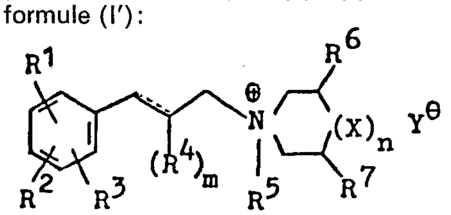
(I')

dans laquelle:

$R^1$, $R^2$ et $R^3$ désignent chacun, indépendamment l'un de l'autre, un atome d'hydrogène, un radical alkyle en $C_1$ à $C_8$, un radical alkyle en $C_1$ à $C_4$ halosubstitué, un groupe phényle éventuellement halo- ou alkyl(en $C_1$ à $C_4$)-substitué, benzyle éventuellement halosubstitué, triméthyl-2,4,6 benzyle, cycloalkyle en $C_3$ à $C_7$, alcoxy en $C_1$ à $C_4$, acyle en $C_2$ à $C_4$ ou halogène,

$R^4$ désigne un atome d'hydrogène, un radical alkyle en $C_1$ à $C_6$, alcényle en $C_3$ ou $C_4$ ou alcoxy en $C_1$ à $C_3$,

$R^5$ désigne un radical alkyle en $C_1$ à $C_4$ ou alcényle en $C_2$ à $C_4$, un groupe propargyle ou un groupe benzyle éventuellement halo- ou alkyl(en $C_1$ à $C_4$)-substitué,

$R^6$ et $R^7$ désignent chacun, indépendamment l'un de l'autre, un atome d'hydrogène, un radical méthyle, éthyle ou propyle, $CH_2OH$ ou OH,

X représente $CH_2$, O, S, C=O, $(CH_2)_2$ ou $CH_2CH-R^8$, où $R^8$ est un radical alkyle,

m vaut 1 ou 2 et n vaut 0 ou 1, et

$Y^{\ominus}$ est l'anion d'un acide non phytotoxique.

6. Procédé de préparation de sels de phénylpropylammonium selon la revendication 1, caractérisé en ce que l'on fait réagir une amine de la formule:

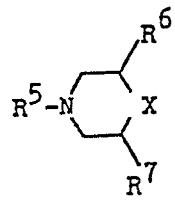

dans laquelle $R^5$, $R^6$, $R^7$ et X possèdent les significations définies dans la revendication 1, éventuellement dans un diluant, avec un composé de la formule:

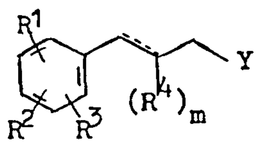

dans laquelle $R^1$, $R^2$, $R^3$, $R^4$ et m possèdent les significations définies dans la revendication 1, et Y représente un atome de chlore, de brome ou d'iode.

**Claims** for the Contracting States BE, CH, DE, FR, GB, IT, LI, NL, SE

1. A phenylpropylammonium salt of the Formula (I)

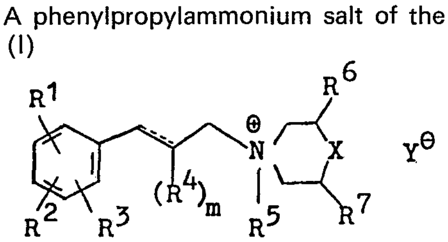
(I)

where $R^1$, $R^2$ and $R^3$ independently of one another are hydrogen, alkyl of 1 to 8 carbon atoms, halogen-substituted alkyl of 1 to 4 carbon atoms, optionally halogen- or $C_1$-$C_4$-alkyl-substituted phenyl, optionally halogen-substituted benzyl, 2,4,6-trimethylbenzyl, cycloalkyl of 3 to 7 carbon atoms, alkoxy of 1 to 4 carbon atoms, acyl of 2 to 4 carbon atoms, or halogen, $R^4$ is hydrogen, alkyl of 1 to 6 carbon atoms, alkenyl of 3 or 4 carbon atoms, or alkoxy of 1 to 3 carbon atoms, $R^5$ is alkyl of 1 to 4 carbon atoms, alkenyl of 4 carbon atoms, propargyl, or optionally halogen- or $C_1$-$C_4$-alkyl-substituted benzyl, $R^6$ and $R^7$ independently of one another are hydrogen, methyl, ethyl, propyl, $CH_2OH$ or OH, X is $CH_2$, O, S, C=O or $CH_2CH-R^8$, $R^8$ being alkyl, m is 1 or 2, and $Y^{\ominus}$ is the anion of a non-phytotoxic acid, except N-n-butyl-3-(3-ethoxyphenyl)propyl-piperidinium oxalate.

2. A phenylpropylammonium salt, as claimed in Claim 1, of the formula

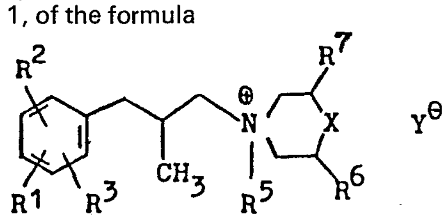

where $R^1$ is alkyl of 1 to 8 carbon atoms or optionally halogen-substituted benzyl, $R^2$ and $R^3$ independently of one another are hydrogen, bromine or chlorine, $R^5$ is alkyl of 1 to 4 carbon atoms, alkenyl of 4 carbon atoms, propargyl, or optionally halogen- or $C_1$-$C_4$-alkyl-substituted benzyl, $R^6$ and $R^7$ independently of one another are hydrogen or methyl, X is O or $CH_2$, and $Y^{\ominus}$ is the anion of a non-phytotoxic acid.

3. A fungicidal agent containing a salt as claimed in Claim 1.

4. A fungicidal agent containing an inert carrier and a salt as claimed in Claim 1.

5. A fungicidal agent containing a salt as claimed in Claim 2.

6. A process for combating fungi, wherein a phenylpropylammonium salt of the Formula (I')

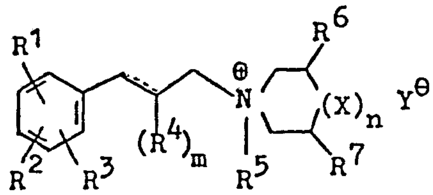

(I')

where $R^1$, $R^2$ and $R^3$ independently of one another are hydrogen, alkyl of 1 to 8 carbon atoms, halogen-substituted alkyl of 1 to 4 carbon atoms, optionally halogen- or $C_1$-$C_4$-alkyl-substituted phenyl, optionally halogen-substituted benzyl, 2,4,6-trimethylbenzyl, cycloalkyl of 3 to 7 carbon atoms, alkoxy of 1 to 4 carbon atoms, acyl of 2 to 4 carbon atoms, or halogen, $R^4$ is hydrogen, alkyl of 1 to 6 carbon atoms, alkenyl of 3 or 4 carbon atoms, or alkoxy of 1 to 3 carbon atoms, $R^5$ is alkyl of 1 to 4 carbon atoms, alkenyl of 2 to 4 carbon atoms, propargyl, or optionally halogen- or $C_1$-$C_4$-alkyl-substituted benzyl, $R^6$ and $R^7$ independently of one another are hydrogen, methyl, ethyl, propyl, $CH_2OH$ or OH, X is $CH_2$, O, S, C=O, $(CH_2)_2$ or $CH_2CH-R^8$, $R^8$ being alkyl, m is 1 or 2, n is 0 or 1, and $Y^\ominus$ is the anion of a non-phytotoxic acid,
is allowed to act on the fungi or on the objects to be protected against fungal attack.

7. A process for the preparation of a phenylpropylammonium salt as claimed in Claim 1, wherein an amine of the formula

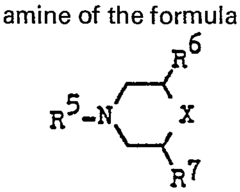

where $R^5$, $R^6$, $R^7$ and X have the meanings given in Claim 1, is reacted, in the presence or absence of a diluent, with a compound of the formula

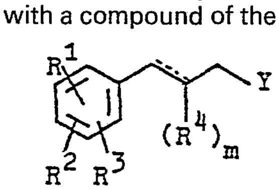

where $R^1$, $R^2$, $R^3$, $R^4$ and m have the meanings given in Claim 1, and Y denotes chlorine, bromine or iodine.

**Claims** for the Contracting State: AT

1. A fungicidal agent containing a phenylpropylammonium salt of the Formula (I)

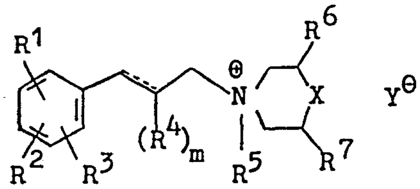

(I)

where $R^1$, $R^2$ and $R^3$ independently of one another are hydrogen, alkyl of 1 to 8 carbon atoms, halogen-substituted alkyl of 1 to 4 carbon atoms, optionally halogen- or $C_1$-$C_4$-alkyl-substituted phenyl, optionally halogen-substituted benzyl, 2,4,6-trimethylbenzyl, cycloalkyl of 3 to 7 carbon atoms, alkoxy of 1 to 4 carbon atoms, acyl of 2 to 4 carbon atoms, or halogen, $R^4$ is hydrogen, alkyl of 1 to 6 carbon atoms, alkenyl of 3 or 4 carbon atoms, or alkoxy of 1 to 3 carbon atoms, $R^5$ is alkyl of 1 to 4 carbon atoms, alkenyl of 4 carbon atoms, propargyl, or optionally halogen- or $C_1$-$C_4$-alkyl-substituted benzyl, $R^6$ and $R^7$ independently of one another are hydrogen, methyl, ethyl, propyl, $CH_2OH$ or OH, X is $CH_2$, O, S, C=O or $CH_2CH-R^8$, $R^8$ being alkyl, m is 1 or 2, and $Y^\ominus$ is the anion of a non-phytotoxic acid,
except N-n-butyl-3-(3-ethoxyphenyl)propylpiperidinium oxalate.

2. A fungicidal agent as claimed in Claim 1, containing a phenylpropylammonium salt of the formula

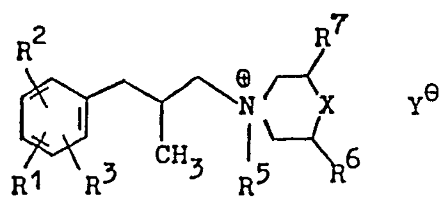

where $R^1$ is alkyl of 1 to 8 carbon atoms or optionally halogen-substituted benzyl, $R^2$ and $R^3$ independently of one another are hydrogen, bromine or chlorine, $R^5$ is alkyl of 1 to 4 carbon atoms, alkenyl of 4 carbon atoms, propargyl, or optionally halogen- or $C_1$-$C_4$-alkyl-substituted benzyl, $R^6$ and $R^7$ independently of one another are hydrogen or methyl, X is O or $CH_2$, and $Y^\ominus$ is the anion of a non-phytotoxic acid.

3. A fungicidal agent containing an inert carrier and a salt as defined in Claim 1.

4. A fungicidal agent containing an inert carrier and a salt as defined in Claim 2.

5. A process for combating fungi, wherein a phenylpropylammonium salt of the Formula (I')

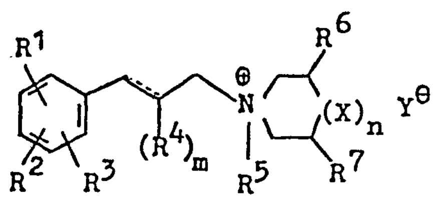

(I')

where $R^1$, $R^2$ and $R^3$ independently of one another are hydrogen, alkyl of 1 to 8 carbon atoms, halogen-substituted alkyl of 1 to 4 carbon atoms, optionally halogen- or $C_1$-$C_4$-alkyl-substituted phenyl, optionally halogen-substituted benzyl, 2,4,6-trimethylbenzyl, cycloalkyl of 3 to 7 carbon atoms, alkoxy of 1 to 4 carbon atoms, acyl of 2 to 4 carbon atoms, or halogen, $R^4$ is hydrogen, alkyl of 1 to 6 carbon atoms, alkenyl of 3 or 4 carbon atoms, or alkoxy of 1 to 3 carbon atoms, $R^5$ is alkyl

of 1 to 4 carbon atoms, alkenyl of 2 to 4 carbon atoms, propargyl, or optionally halogen- or $C_1$-$C_4$-alkyl-substituted benzyl, $R^6$ and $R^7$ independently of one another are hydrogen, methyl, ethyl, propyl, $CH_2OH$ or OH, X is $CH_2$, O, S, C=O, $(CH_2)_2$ or $CH_2CH-R^8$, $R^8$ being alkyl, m is 1 or 2, n is 0 or 1, and $Y^\ominus$ is the anion of a non-phytotoxic acid,
is allowed to act on the fungi or on the objects to be protected against fungal attack.

6. A process for the preparation of a phenylpropylammonium salt as defined in Claim 1, wherein an amine of the formula

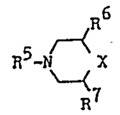

where $R^5$, $R^6$, $R^7$ and X have the meanings given in Claim 1, is reacted, in the presence or absence of a diluent, with a compound of the formula

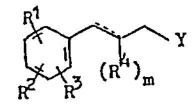

where $R^1$, $R^2$, $R^3$, $R^4$ and m have the meanings given in Claim 1, and Y denotes chlorine, bromine or iodine.